# FASCICULE DE BREVET EUROPEEN

(11) **EP 4 227 575 B1**
(45) Date de publication et mention de la délivrance du brevet: **18.12.2024**
(21) Numéro de dépôt: 23153708.5
(22) Date de dépôt: 27.01.2023
(51) Int. Cl.: A61M 16/00, F17C 11/00, A61M 16/10, A61M 16/20, A61M 16/22, A61M 16/06, A61M 16/08, A62B 7/02, A62B 7/08, A62B 21/00, B01D 53/04

(54) **ENSEMBLE PORTATIF DE DISTRIBUTION D OXYGÈNE DE SECOURS COMPRENANT UNE CARTOUCHE DE STOCKAGE D OXYGÈNE**
TRAGBARE NOTSAUERSTOFFABGABEANORDNUNG MIT SAUERSTOFFSPEICHERPATRONE
PORTABLE EMERGENCY OXYGEN DELIVERY ASSEMBLY INCLUDING OXYGEN STORAGE CARTRIDGE

(30) Priorité: 11.02.2022 FR 2201224
(43) Date de publication de la demande: 16.08.2023
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: BOULANGER, Thierry, 75007 Paris (FR); PATEL, Akshar, 75007 Paris (FR)
(74) Mandataire: Air Liquide

(56) Documents cités:
- US-A- 2 944 547
- US-A1- 2010 089 237
- US-A1- 2011 041 519
- US-A1- 2016 201 853

## Description

La présente invention concerne un ensemble de distribution d'oxygène de secours, en particulier un ensemble portatif, comprenant une cartouche de stockage d'oxygène raccordée fluidiquement à un réservoir de gaz et à une interface respiratoire, permettant une fourniture d'oxygène (O₂) à un utilisateur, pendant un temps suffisant, dans une situation particulière engendrant une atmosphère néfaste, hypoxique et/ou chargée de composés délétères, en particulier en présence de fumées générées par un incendie.

L'administration d'O₂ gazeux sert normalement à corriger une situation hypoxémique, c'est-à-dire lorsque la saturation d'oxyhémoglobine dans le sang est inférieure à une valeur normale, par exemple inférieure à 90%, chez une personne humaine, quel que soit son âge, i.e. nouveaux nés, enfants, adolescents, adultes, personnes âgées ou autres, souffrant une pathologie respiratoire du type Bronchopneumopathie Chronique Obstructive (BPCO), Syndrome de Détresse Respiratoire Aigüe (SDRA) ou autre.

Dans ce cas, l'oxygène de qualité médicale est fourni à la personne « hypoxique » soit au sein d'un l'hôpital, soit hors hôpital, par exemple dans une unité de secours mobile (SAMU, ambulances...) ou au domicile du patient.

Selon le cas, l'oxygène fourni au patient peut provenir soit d'une canalisation de gaz (e.g. réseau hospitalier), soit d'une bouteille de gaz sous pression contenant par exemple de 400 à 1000 L d'O₂ (vol. gazeux) comprimé jusqu'à 200 bar abs ou plus (mesuré à 1 atm).

Il existe toutefois des situations particulières dans lesquelles un apport d'O₂ est nécessaire, avant même qu'une personne ne montre des signes d'hypoxémie. C'est en particulier le cas lorsqu'une personne est exposée à une atmosphère gazeuse « irrespirable », par exemple en présence de fumées dégagées par un incendie ou en cas de fuite de composés chimiques volatils au sein d'une pièce ou d'un bâtiment.

Dans une telle situation particulière, l'air ambiant est souvent chargé en composés néfastes, telles des particules fines, du monoxyde de carbone (CO) et d'autres particules organiques volatiles plus ou moins toxiques pour une personne qui serait amenée à les inhaler.

De plus, l'air présente souvent une teneur en oxygène insuffisante, c'est-à-dire inférieure à la quantité d'oxygène normalement présent dans l'air ambiant (i.e. <21% vol.), résultant par exemple de la combustion de divers matériaux en cas d'incendie. Ceci explique pourquoi plus de 80% des victimes d'un incendie succombent de suffocations du fait de l'inhalation d'une atmosphère « irrespirable », et non pas de brûlures occasionnées par les flammes.

Or, les sources d'oxygène de secours classiques (i.e. canalisation de gaz ou bouteille de gaz) ne sont pas utilisables ou pas adaptées à de telles situations particulières.

Ainsi, on comprend aisément qu'il n'est pas possible d'équiper de nombreux bâtiments de canalisations d'oxygène.

Par ailleurs, en théorie, on pourrait disposer des bouteilles d'oxygène de secours dans les bâtiments ou bien en équiper les secouristes, tels les pompiers, afin de leur permettre de donner une autonomie respiratoire suffisante aux victimes, leur permettant de s'échapper des lieux d'un incendie ou autre.

Toutefois, cette solution n'est pas réaliste et/ou difficilement applicable en pratique.

Tout d'abord, les récipients d'oxygène sous haute pression (i.e. 200 bar ou plus), telles les bouteilles d'oxygène, pour des raisons réglementaires, ne peuvent être librement laissés dans des lieux accessibles au public, notamment du fait des risques de vols, d'explosion et de mésusage du gaz par des tiers non formés à la distribution des gaz, y compris les victimes elles-mêmes.

Par ailleurs, une bouteille de gaz pleine d'oxygène et munie d'un détendeur de gaz, voire d'un capotage de protection, est relativement encombrante et pèse plusieurs kg, typiquement d'au moins 5 à 10 kg, ce qui limite leur mobilité et la capacité d'un secouriste à en transporter plusieurs afin de les distribuer à toutes les victimes d'un incendie ou autre.

US2944547 propose un système de fourniture de gaz en particulier pour un patient.

US-A-20210121649 propose un système de fourniture de gaz composé d'une bouteille d'oxygène à haute pression munie d'un détendeur de gaz, d'une tubulure alimentant un réservoir de gaz et un module contenant de la chaux pour épurer les gaz expirés, de sorte d'obtenir un circuit fermé de gaz. Cependant, un tel système n'est pas non plus idéal puisqu'il se heurte aux mêmes limitations que ci-dessus.

Un problème est dès lors de pouvoir fournir un apport d'oxygène de secours à une personne confrontée à situation particulière engendrant une atmosphère néfaste hypoxique (i.e. <21% O₂) et/ou chargée de composés délétères, comme les fumées en cas d'incendie ou analogue, et ce, de manière simple et efficace, pour lui permettre d'éviter de respirer cette atmosphère néfaste et sans rencontrer les inconvénients et contraintes susmentionnés, de manière à pouvoir lui fournir de l'oxygène pendant une durée de plusieurs dizaines de minutes.

Une solution de l'invention concerne un ensemble de distribution d'oxygène de secours, en particulier un ensemble portatif, comprenant une cartouche de stockage d'oxygène comprenant un corps tubulaire fermé à deux extrémités opposées et délimitant un volume interne servant au stockage de l'oxygène.

De plus :
- au moins une valve de contrôle de flux gazeux est agencée à chacune des deux extrémités, et
- le volume interne comprend une chambre centrale contenant au moins un lit d'adsorbant, une première chambre de recueil de gaz et une seconde chambre de recueil de gaz, la chambre centrale étant située entre les première et seconde chambres de recueil de gaz.

La cartouche de l'ensemble de distribution d'oxygène de secours est raccordée fluidiquement à un réservoir de gaz et à une interface respiratoire.

Selon le mode de réalisation considéré, l'ensemble de distribution d'oxygène de secours de l'invention, notamment la cartouche, peut comprendre l'une ou plusieurs des caractéristiques suivantes :
- le corps tubulaire est fermé à ses deux extrémités opposées par un premier et un second couvercle, c'est-à-dire à une première et une seconde extrémité.
- au moins une valve de contrôle de flux gazeux est agencée sur chacun desdits premier et second couvercles.
- une première valve de contrôle de flux gazeux est agencée à la première des deux extrémités opposées, de préférence sur le premier couvercle, c'est-à-dire que la première valve est portée par le premier couvercle.
- une seconde valve de contrôle de flux gazeux est agencée à la seconde des deux extrémités opposées, de préférence sur le second couvercle, c'est-à-dire que la seconde valve est portée par le second couvercle.
- les première et seconde valves de contrôle sont identiques ou différentes.
- le volume interne comprend en outre une première chambre de recueil de gaz et une seconde chambre de recueil de gaz, la chambre centrale étant située entre la première et la seconde chambre de recueil de gaz.
- la première valve de contrôle de flux gazeux est en communication fluidique avec la première chambre de recueil de gaz lorsque la première valve de contrôle est en position ouverte, c'est-à-dire que la première valve de contrôle est configurée pour mettre en relation fluidique l'intérieur et l'extérieur du corps tubulaire de la cartouche, lorsqu'elle est ouverte.
- la seconde valve de contrôle de flux gazeux est en communication fluidique avec la seconde chambre de recueil de gaz lorsque la seconde valve de contrôle est en position ouverte c'est-à-dire que la seconde valve de contrôle est configurée pour mettre en relation fluidique l'intérieur et l'extérieur du corps tubulaire de la cartouche, lorsqu'elle est ouverte.
- la première valve de contrôle de flux gazeux est configurée pour laisser un flux gazeux la traverser lorsqu'elle est en position ouverte.
- la seconde valve de contrôle de flux gazeux est configurée pour laisser un flux gazeux la traverser lorsqu'elle est en position ouverte.
- la chambre centrale de la cartouche est séparée de la première et de la seconde chambre de recueil de gaz par une première et une seconde structure de tamis, respectivement.
- les première et seconde structure de tamis comprennent des grilles ou analogues.
- les grilles ont une forme de disque.
- le corps tubulaire de la cartouche a une section interne et les structures de tamis sont agencées radialement dans le volume interne, i.e. perpendiculairement à l'axe principal (XX), de manière à s'étendre sur toute la section interne du corps tubulaire.
- le corps tubulaire de la cartouche est cylindrique.
- les structures de tamis sont configurées pour maintenir le lit d'adsorbant.
- le lit d'adsorbant contient des particules de zéolite, de préférence une zéolite 13X ou 5A présentant une capacité d'adsorption pour l'oxygène d'au moins 3 ml/g à 1 bar et 20°C.
- chaque valve de contrôle de la cartouche comprend un corps de valve, un moyen d'étanchéité annulaire, un élément mobile et un moyen élastique coopérant les uns avec les autres pour contrôler le passage de gaz au travers de chaque valve de contrôle.
- chaque valve de contrôle de la cartouche comprend en outre une pièce-couvercle venant se fixer au corps de valve.
- la pièce-couvercle de la cartouche vient se fixer par vissage au corps de valve de chaque valve de contrôle.
- la pièce-couvercle de la cartouche porte un filetage périphérique externe et le corps de valve de chaque valve de contrôle porte un taraudage interne complémentaire du filetage périphérique externe de la pièce-couvercle.
- chaque valve de contrôle est mobile entre une position de repos et une position actionnée ou active, i.e. ouverte.
- en position de repos ou fermée, aucun gaz ne peut entrer ou sortir de la cartouche par l'une et/ou l'autre des valves de contrôle.
- en position actionnée ou active, un flux de gaz peut s'établir au travers de l'une et/ou l'autre des valves de contrôle de manière entrer ou sortir de la cartouche via l'une et/ou l'autre des valves de contrôle.
- le moyen élastique est agencé dans la pièce-couvercle.
- le moyen élastique est pris en sandwich entre la pièce-couvercle et l'élément mobile.
- le moyen élastique est maintenu par des bossages agencés sur le fond de la pièce-couvercle et sur la paroi extérieure du fond de l'élément mobile.
- l'élément mobile comprend une collerette périphérique.
- le moyen élastique repousse l'élément mobile contre le moyen d'étanchéité annulaire.
- le moyen élastique repousse l'élément mobile contre le moyen d'étanchéité annulaire de sorte que la collerette périphérique de l'élément mobile vienne au contact du moyen d'étanchéité lorsqu'une valve est en position de repos.
- le moyen élastique est un ressort à spires cylindrique.
- le moyen d'étanchéité annulaire est un joint torique plat.
- le corps de valve comprend un fond annulaire présentant un évidement central, le moyen d'étanchéité annulaire étant agencé sur ledit fond annulaire, coaxialement audit évidement central.
- l'élément mobile comprend une collerette coopérant avec le moyen d'étanchéité annulaire pour assurer une étanchéité fluidique et empêcher toute circulation de gaz au travers de l'évidement central du corps de valve de l'une et/ou l'autre des valves de contrôle, lorsque l'une et/ou l'autre des valves de contrôle est/sont en position de repos.
- en position active, la collerette de l'élément mobile n'est pas ou plus au contact du moyen d'étanchéité annulaire de sorte que étanchéité fluidique soit rompue et qu'une circulation de gaz au travers de l'évidement central du corps de valve de l'une et/ou l'autre des valves de contrôle puisse s'établir.
- le corps de valve, le moyen d'étanchéité annulaire, l'élément mobile, le moyen élastique et la pièce-couvercle de chaque valve de contrôle sont coaxiaux (axe XX).
- le corps de valve de chaque valve de contrôle a une forme tubulaire, typiquement cylindrique.
- le corps de valve comprend une collerette annulaire se projetant radialement vers l'extérieur.
- la collerette annulaire est agencée au niveau d'une première extrémité du corps de valve.
- le corps de valve de chaque valve de contrôle comprend intérieurement un fond annulaire, de préférence plat.
- le fond annulaire du corps de valve présente en son centre, un évidement circulaire.
- le fond annulaire du corps de valve forme un épaulement interne s'étirant le long de la paroi interne, i.e. du périmètre interne, du corps de valve.
- l'élément mobile est mobile en translation axiale (axe XX) dans le corps de valve de chaque valve.
- l'élément mobile a une forme de coupelle.
- l'élément mobile comprend une ou plusieurs ouvertures aménagées dans sa paroi périphérique.
- la pièce-couvercle a une forme de coupelle.
- la pièce-couvercle comprend une ou plusieurs ouvertures aménagées dans sa paroi périphérique.
- la paroi périphérique de la pièce-couvercle a une forme cylindrique.
- l'élément mobile de chaque valve de contrôle comprend une collerette périphérique externe comprenant un ou plusieurs logements ou évidements, telles des encoches.
- la pièce-couvercle comprend un ou des guides axiaux, tels des rails de guidage ou analogues, aménagés axialement le long de sa paroi interne.
- le ou les évidements portés par la collerette périphérique externe de l'élément mobile sont conformés pour loger les guides axiaux de la pièce-couvercle de sorte que ces guides axiaux puissent guider un déplacement translatif de la pièce mobile dans la pièce-couvercle.
- les couvercles prennent le corps cylindrique en « sandwich », c'est-à-dire qu'ils sont situés aux extrémités opposées du corps cylindrique.
- les couvercles sont fixés de manière étanche au corps tubulaire de la cartouche, typiquement au corps cylindrique, par exemple soudés ou sertis.
- les couvercles et le corps tubulaire de la cartouche sont en alliage d'aluminium.
- le réservoir de gaz comprend une enveloppe flexible, en particulier en polymère.
- l'interface respiratoire comprend un masque respiratoire, tel un masque nasal ou facial.
- la cartouche est raccordée fluidiquement à un réservoir de gaz à enveloppe flexible, par exemple en polymère.
- la cartouche est raccordée fluidiquement à une interface respiratoire de type masque respiratoire, tel un masque nasal ou facial.
- le masque respiratoire comprend un corps de masque rigide portant un coussinet flexible destiné à venir au contact du visage d'un utilisateur pour assurer une étanchéité gazeuse et un confort d'utilisation.
- la cartouche comprend des moyens de raccordement configurés pour permettre d'y raccorder fluidiquement le réservoir et l'interface respiratoire, typiquement de manière étanche.
- les moyens de raccordement sont portés par les premier et second couvercles.
- les moyens de raccordement sont configurés pour permettre un raccordement par clipsage, vissage, emboitement, système à baïonnette ou autre.
- l'interface respiratoire et le réservoir de gaz comprennent chacun un conduit ou embout de raccordement, en particulier un premier conduit et un second conduit.
- le premier conduit et le second conduit comprennent chacun une enveloppe cylindrique comprenant un conduit interne creux ou lumen, i.e. ayant une forme de cylindre creux.
- les premier et second conduits viennent coopérer avec les moyens de raccordement de la cartouche pour permettre un raccordement fluidique de l'interface respiratoire et du réservoir de gaz au corps de cartouche.
- selon un mode de réalisation, les moyens de raccordement de la cartouche peuvent comprendre des taraudages pour permettre un raccordement par vissage avec des filetages portés par les premier et second conduits du réservoir et de l'interface respiratoire.
- selon un autre mode de réalisation, les moyens de raccordement de la cartouche et les premier et second conduits du réservoir et de l'interface respiratoire peuvent avoir des structures complémentaires de type mâle/femelle pour assurer un raccordement ou assemblage par emboitement ou analogue.
- selon encore un autre mode de réalisation, les moyens de raccordement de la cartouche et les premier et second conduits du réservoir et de l'interface respiratoire peuvent être configurés pour permettre un assemblage ou raccordement de type baïonnette, c'est-à-dire porter un système à baïonnette.
- le premier conduit vient se raccorder en regard de la première valve de manière à assurer une continuité fluidique entre eux.
- le second conduit vient se raccorder en regard de la seconde valve de manière à assurer une continuité fluidique entre eux.
- la cartouche a un volume intérieur compris entre 200 et 1000 ml, avantageusement compris entre 300 ml à 900 ml (équiv. en eau).
- le réservoir est configuré pour collecter un volume de gaz supérieur ou égal au volume de gaz contenu dans le volume intérieur de la cartouche de gaz.
- le réservoir a un volume interne compris entre 500 et 50 000 ml (équiv. en eau).
- la cartouche a un volume intérieur adapté au stockage d'oxygène gazeux.

En outre, l'invention concerne aussi l'utilisation d'un ensemble de distribution d'oxygène de secours selon l'invention pour fournir de l'oxygène à une personne.

L'invention va maintenant être mieux comprise grâce à la description détaillée suivante, faite à titre illustratif mais non limitatif, en référence aux figures annexées parmi lesquelles :
Fig. 1 est une vue schématique en coupe d'un mode de réalisation d'une cartouche de stockage d'oxygène selon l'invention,
Fig. 2 est une vue éclatée d'une des valves intégrées à la cartouche de stockage d'oxygène de Fig. 1,
Fig. 3 est une vue éclatée d'une partie de la valve de Fig. 2,
Fig. 4 est une vue latérale et en coupe de la valve de Fig. 2, dans sa position de « repos »,
Fig. 5 est une vue en coupe de la valve de Fig. 2, dans sa position « actionnée »,
Fig. 6 est une vue latérale de la valve de Fig. 5,
Fig. 7 illustre la cartouche de stockage d'oxygène de Fig. 1, avec ses valves en position « actionnée »,
Fig. 8 schématise la cartouche de stockage d'oxygène de Fig. 1, raccordée à un réservoir (partiellement montré) et à un masque (non montré), via des conduits de gaz, et
Fig. 9 schématise un ensemble de distribution d'oxygène de secours comprenant la cartouche de stockage d'oxygène de Fig. 1, un réservoir pour l'oxygène et une interface respiratoire.

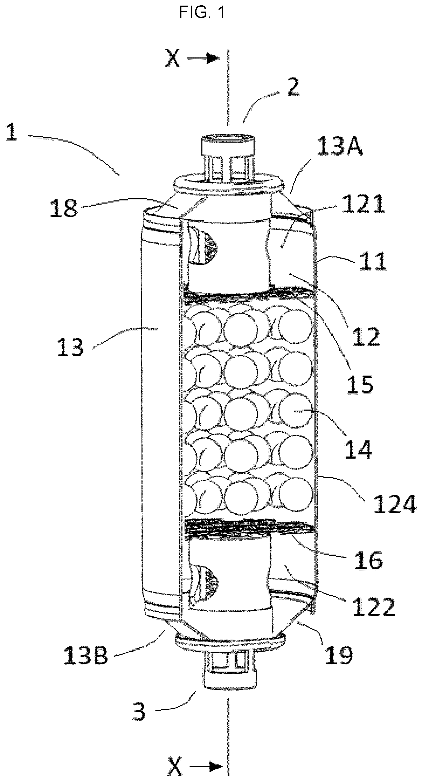

Fig. 1 est une vue schématique en coupe d'un mode de réalisation d'une cartouche 1 de fourniture d'oxygène de secours selon l'invention.

La cartouche 1 comprend un corps principal 13 fermé à ses deux extrémités opposées 13A, 13B par un premier couvercle 18 et un second couvercle 19, telles des parois ou analogues fermant des deux extrémités 13A, 13B du corps principal 13, de manière à délimiter un volume interne 12 servant au stockage du gaz, à savoir ici de l'oxygène (O₂) sous forme gazeuse.

Le corps principal 13 est formé ici d'une enveloppe tubulaire 11 externe, préférentiellement de forme cylindrique. Le corps principal 13 est de préférence en métal, par exemple en un alliage d'aluminium. L'enveloppe 11 tubulaire est allongée et s'étend selon un axe principal XX entre les deux extrémités 13A, 13B.

Le premier couvercle 18 et le second couvercle 19 prennent le corps cylindrique 13, typiquement l'enveloppe 11 tubulaire, en « sandwich » et y sont fixés de manière étanche, par exemple soudés ou sertis.

Les premier et un second couvercle 18, 19 sont préférentiellement aussi en métal, par exemple en alliage d'aluminium.

La paroi de l'enveloppe périphérique 11 formant le corps principal 13 est fine, c'est-à-dire qu'elle a une épaisseur de l'ordre de quelques dixièmes de millimètres.

Par ailleurs, le volume intérieur de la cartouche 1 est de l'ordre de 300 ml à 900 ml (équiv. en eau).

Un (ou plusieurs) lit(s) d'un (ou plusieurs) adsorbant particulaire 14, telles des billes d'adsorbant par exemple, est agencé dans le volume intérieur 12. En particulier, le lit d'adsorbant 14 est pris en sandwich et maintenu par/entre deux structures de tamis 15, 16, plus simplement appelées 'tamis', c'est-à-dire que le premier tamis 15 et le second tamis 16 sont disposés de part et d'autre du lit d'adsorbant 14 de manière à le maintenir en position dans le volume interne 12 de la cartouche 1.

Autrement dit, les structures de tamis 15, 16 qui sont agencées radialement dans le volume interne 12, c'est-à-dire perpendiculairement à l'axe principal XX, et s'étendent sur toute la section interne du corps principal 13 de forme cylindrique.

Préférentiellement, les structures de tamis 15, 16 permettent de séparer le volume interne 12 en plusieurs chambres ou compartiments internes, à savoir une chambre centrale 124 contenant le lit d'adsorbant 14 située au centre de la cartouche 1, et une première et une seconde chambre de recueil de gaz 121, 122 situées aux extrémités opposées 13A, 13B, c'est-à-dire entre la chambre centrale 124 et les premier et second couvercle 18, 19.

Les tamis 15, 16 sont par exemple formés d'un maillage de fibres métalliques présentant une multitude de pores dont les dimensions sont inférieures aux dimensions de l'adsorbant 14. Selon d'autres modes de réalisation, les tamis 15, 16 peuvent se présenter sous forme de mousses comprenant ou formées d'alvéoles interconnectées, ou encore sous forme de grilles, par exemple de grilles métalliques, ou analogues.

Dans tous les cas, les tamis 15, 16 sont dimensionnés pour retenir les particules d'adsorbant du lit d'adsorbant 14. Autrement dit, quel que soit le mode de réalisation choisi, le lit d'adsorbant 14 est « piégé » et maintenu entre les deux tamis 15, 16, alors que les molécules de gaz sont libres de circuler à travers eux.

En outre, l'adsorbant 14 est choisi pour permettre d'adsorber les molécules de gaz, en particulier celles d'oxygène, du fait de minuscules pores à sa surface, c'est-à-dire des pores de l'ordre du ou de quelques nanomètre (nm). Ainsi, il peut être choisi parmi les charbons actifs, les structures organométalliques ou MOF pour *« Metal Organic Framework* » ou les zéolites. Toutefois, on utilise, de préférence, un adsorbant zéolitique se présentent sous forme de particules, telles des granules, des billes ou analogues, d'une zéolite ayant une capacité d'adsorption élevée, c'est-à-dire d'au moins 3 ml/g à 1 bar et 20°C, pour l'oxygène, par exemple une zéolite 13X ou 5A, par exemple la zéolite référencée Z10-ZZ commercialisée par la société Zeochem^{®}.

Comme visible sur Fig. 1, le lit d'adsorbant 14 ne remplit pas l'intégralité du volume interne 12 de la cartouche 1 mais est agencé uniquement dans la chambre centrale 124 de sorte qu'il existe des espaces vides aux deux extrémités 13A, 13B du volume interne 12 formant les première 121 et seconde 122 chambres de recueil de gaz.

La première chambre 121 de recueil de gaz est située entre le premier couvercle 18 et le premier tamis 15, et la seconde chambre 122 de recueil de gaz est située entre le second couvercle 19 et le second tamis 16. Autrement dit, les deux tamis 15, 16 séparent la chambre centrale 124 contenant le lit d'adsorbant 14, des première et seconde chambres de recueil de gaz 121, 122 qui sont situées de part et d'autre de ladite chambre centrale 124, c'est-à-dire qui la prennent en sandwich en étant séparées de celle-ci par les tamis 15, 16.

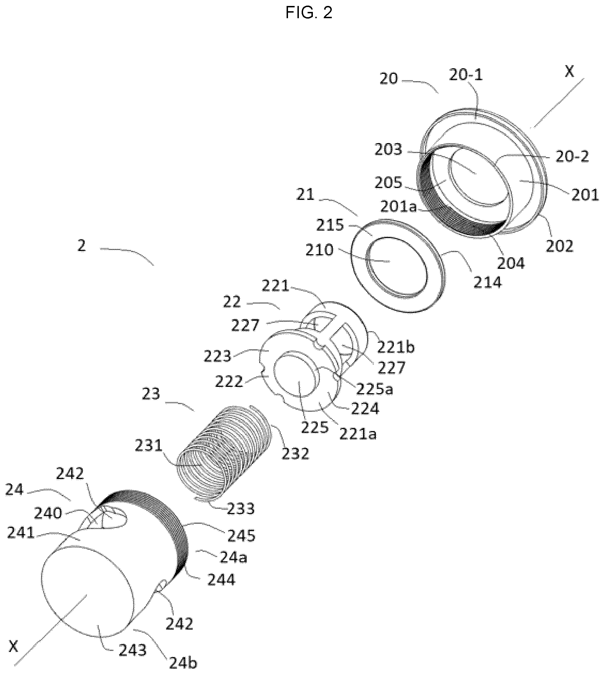

De plus, comme visible sur Fig. 1, la cartouche 1 d'oxygène gazeux selon l'invention présente aussi une première valve 2 et une seconde valve 3 de contrôle de flux agencées à ses deux extrémités opposés 13A, 13B, typiquement portées par les couvercles 18, 19. Les première et seconde valves de contrôle de flux 2, 3 servent à contrôler la circulation de gaz, c'est-à-dire les flux gazeux entrant et sortant du volume interne 12 de la cartouche 1, comme expliqué ci-après et illustré sur Fig. 2 à Fig. 9. Elles sont fixées de manière étanche, par exemple soudées ou serties, aux couvercles 18, 19, typiquement elles traversent les couvercles 18, 19.

Les première et seconde valves 2, 3 de contrôle de flux sont préférentiellement mais pas obligatoirement identiques.

Un mode de réalisation de l'architecture de ces valves 2, 3 est détaillé ci-après en lien avec Fig. 2 à Fig. 7.

Fig. 2 est vue éclatée de la première valve 2 équipant la cartouche 1 de stockage d'oxygène de l'invention schématisée en Fig. 1, sachant que la seconde valve 3 lui est strictement identique et fonctionne de la même façon (la valve 3 n'est donc pas détaillée ci-après).

Plus précisément, la première valve 2, tout comme la deuxième valve 3, est formée d'un assemblage de plusieurs éléments 20-24 coopérant les uns avec les autres, comprenant un corps de valve 20, un moyen d'étanchéité annulaire 21, un élément mobile 22, un moyen élastique 23, tel un ressort cylindrique à spires, et une pièce-couvercle 24, qui sont détaillés ci-après.

Ces éléments 20-24 sont agencés coaxialement les uns par rapport aux autres par rapport à l'axe XX des valves 2, 3 et de la cartouche 1. De préférence, les éléments 20-24 sont métalliques, par exemple en un alliage d'aluminium ; bien entendu, d'autres matériaux adaptés peuvent aussi convenir.

Le corps de valve 20 est de forme générale cylindrique. Il a une longueur de l'ordre de 8 à 16 mm mesurée entre ses deux extrémités 20-1, 20-2, et un diamètre interne (Di) typiquement compris entre 18 et 30 mm.

La paroi périphérique 201 du corps de valve 20 porte, au niveau de sa première extrémité 20-1, une collerette annulaire 202 se projetant radialement vers l'extérieur.

Autrement dit, la collerette annulaire 202 est solidaire de la paroi périphérique 201 externe cylindrique du corps de valve 20. Elles peuvent être formées d'une seule pièce ou de plusieurs pièces assemblées les uns aux autres.

La collerette 202 forme un épaulement externe, s'étirant sur toute la périphérie externe ou périmètre du corps de valve 20, permettant d'assurer une étanchéité fluidique entre le corps de valve 20 et le couvercle 18 intégrant la première valve 2, par exemple en y étant fixé par sertissage, soudage ou toute autre technique adaptée.

Par ailleurs, le corps de valve 20 présente aussi intérieurement un fond annulaire 205, de préférence plat. Le fond annulaire 205 présente en son centre, un évidement circulaire 203, et forme un épaulement interne, s'étirant le long de la paroi interne ou périmètre interne du corps de valve 20.

De plus, le corps de valve 20 porte un taraudage 204 aménagé sur une partie 201a de sa paroi interne 201, à partir de sa seconde extrémité 20-2. Ce taraudage 204 permet le raccordement par vissage de l'élément support 24, comme expliqué ci-après.

La première valve 2 comprend par ailleurs un moyen d'étanchéité annulaire 21, à savoir ici un joint annulaire plat, e.g. un joint torique.

De préférence, il a une épaisseur d'environ 1 mm et un diamètre externe (De) légèrement inférieur au diamètre interne (Di) du corps de valve 20, i.e. De<Di, de manière à pouvoir être inséré dans le corps de valve 20 et venir se positionner sur l'épaulement interne formé par le fond annulaire 205 du corps de valve 20.

Le joint annulaire plat 21 comprend une face avant 214 orientée vers le fond annulaire 205 du corps de valve 20 et une face arrière 215 opposée à la face avant 214, c'est-à-dire orientée en direction de la collerette 222 de l'élément mobile 22, comme détaillé ci-après. De plus, il présente par ailleurs un passage central 210 dont le diamètre est supérieur à celui de l'évidement circulaire 203 du corps de valve 20.

Il peut être constitué de différents matériaux, en particulier un élastomère, par exemple le caoutchouc nitrile, aussi appelé caoutchouc acrylonitrile butadiène ou caoutchouc NBR pour « Nitrile Butadiene Rubber » en anglais.

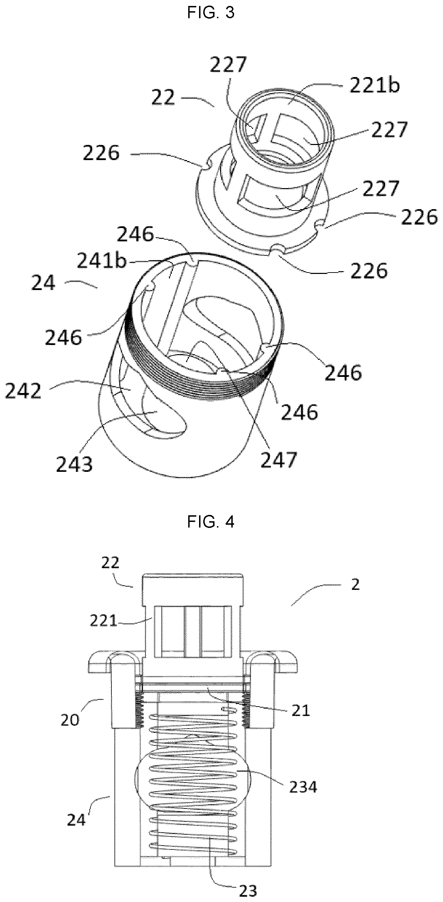

Comme visible sur Fig. 2 et Fig. 3, l'élément mobile 22 est une structure ayant une forme générale de coupelle. Plus précisément, l'élément mobile 22 comprend une portion avant 221 de forme cylindrique qui est ouverte à son extrémité distale 221b et obturée, c'est-à-dire fermée, à son extrémité proximale 221a par une paroi de fond 223, par exemple en forme de disque.

Préférentiellement, la paroi de fond 223 se projette radialement vers l'extérieur, c'est-à-dire en éloignement par rapport à la surface périphérique externe de la portion avant 221 de forme cylindrique de manière à former une collerette 222, i.e. une structure en anneau, s'étendant tout autour de l'extrémité proximale 221a cylindrique de la portion avant 221.

La portion cylindrique 221a un diamètre externe inférieur au diamètre interne de l'évidement circulaire 203 du corps de valve 20, alors que la collerette 222 a un diamètre externe supérieur à celui de l'évidement 203 du corps de valve 20 de sorte que l'élément mobile 22 soit mobile axialement (axe XX) au travers de l'évidement circulaire 203 du corps de valve 20 et de l'évidement central 210 du joint annulaire plat 21 mais ne puisse pas totalement en sortir, étant donné qu'il y est retenu par la collerette 222 lorsque celle-ci vient buter sur le joint annulaire plat 21, qui est luimême agencé sur le fond annulaire 205 du corps de valve 20, c'est-à-dire pris en sandwich entre le fond annulaire 205 du corps de valve 20 et la collerette 222 de l'élément mobile 22.

La paroi périphérique de la portion avant 221 cylindrique est ajourée, c'est-à-dire qu'elle présente plusieurs ouvertures latérales 227.

Par ailleurs, la face extérieure 224 de la paroi de fond 223 comprend un bossage central 225, c'est-à-dire un élément en relief, une excroissance de surface ou analogue, typiquement de section circulaire. Ici, le bossage central 225 est de forme générale cylindrique et a une hauteur de l'ordre de 1 mm et un diamètre qui est inférieur au diamètre de la collerette 222, comme visible en Fig. 2, par exemple un diamètre de l'ordre de 4 à 8 mm. Lorsque le moyen élastique 23 est un ressort à spires cylindrique délimitant un passage central cylindrique 231, le diamètre du bossage central 225 est légèrement inférieur à celui du passage central 231 du ressort de manière à ce que le ressort puisse venir s'insérer sur le bossage central 225 à la manière d'un manchon. Le bossage central 225 coopère avec le moyen élastique 23, comme expliqué ci-après.

Le moyen élastique 23 est préférentiellement un ressort à spires cylindrique venant se positionner autour du bossage central 225, tel un manchon, et appuyer sur la face extérieure 224 de la paroi de fond 223 dans une zone sensiblement annulaire située immédiatement autour du bossage central 225 de l'élément mobile 22. Le moyen élastique 23, i.e. le ressort, comprend une extrémité avant 232 venant au contact de l'élément mobile 22 et une extrémité arrière 233 venant au contact de pièce-couvercle 24.

Le diamètre interne du passage central 231 du ressort à spires est dès lors légèrement supérieur au diamètre du bossage central 225, comme déjà expliqué.

Le moyen élastique 23 repousse élastiquement l'élément mobile 22 en direction du moyen d'étanchéité 21.

La première valve 2 comprend par ailleurs une pièce-couvercle 24 tubulaire délimitant un logement interne 240 pour loger (au moins) le moyen élastique 2 qui est comprimé et va alors repousser axialement (XX) l'élément mobile 22.

Comme visible sur Fig. 2 et Fig. 3, la pièce-couvercle 24 est formée d'un corps principal 241 de forme cylindrique dont la paroi est percée, c'est-à-dire traversée, par une ou des ouvertures ou fenêtres latérales 242. Le corps principal 241 comprend, à son extrémité avant 24a, une ouverture 245 qui communique avec le logement interne 240. A son extrémité arrière 24b, le corps principal est fermé par une paroi formant un fond 243, de préférence un fond plat. Autrement dit, la pièce-couvercle 24 a une forme générale de coupelle ou similaire avec des ouvertures latérales 242.

Le corps principal 241 de la pièce-couvercle 24 présente par ailleurs, sur sa surface extérieure, du côté de son extrémité avant 24a, un filetage 244 périphérique qui est complémentaire du taraudage 204 du corps de valve 20 pour que la pièce couvercle 24 puisse venir se fixer par vissage audit corps de valve 20 en prenant en 'sandwich' le moyen élastique 23, l'élément mobile 22 et le moyen d'étanchéité 21, comme visible sur Fig. 4.

Comme visible sur Fig. 2 et Fig. 4, le moyen d'étanchéité, c'est-à-dire le joint plat 21, est inséré dans le corps de valve 20. Sa face avant 214 vient en contact avec le fond annulaire 205 qui forme un épaulement interne du corps de valve 20.

L'évidement circulaire 203 situé au centre du fond annulaire 205 du corps de valve 20 est en communication fluidique avec le passage central 210 du joint plat 21.

Comme illustré en Fig. 4, lorsque la pièce-couvercle 24 est visée sur le corps de valve 20, elle agit sur le moyen élastique 23, à savoir le ressort à spires cylindrique, qui est alors comprimé et repousse alors, à son tour, l'élément mobile 22 en direction du corps de valve 20. L'élément mobile 22 se déplace alors axialement jusqu'à ce que sa portion avant 221 de forme cylindrique passe au travers du passage central 210 du joint plat 21 et de l'évidement circulaire 203 situé au centre du fond annulaire 205 du corps de valve 20 jusqu'à faire saillie hors du corps de valve 20. Le mouvement translatif de l'élément mobile 22 est stoppé lorsque la collerette 222 vient en butée contre la face arrière 215 du joint plat 21.

Le joint plat 21 se retrouve alors pris en « sandwich » entre le fond plat 205 du corps de valve 1 et la collerette 222 de la pièce mobile 22 qui est repoussée par le ressort cylindrique 23.

Plus précisément, l'extrémité avant 232 du ressort cylindrique 23 vient en contact avec la face extérieure 224 de la paroi de fond 223 de l'élément mobile 22 pour le repousser élastiquement axialement vers le joint plat 21, c'est-à-dire en direction du corps de valve 1.

L'extrémité arrière 233 du ressort cylindrique 23 est quant à elle en contact avec la surface interne de la paroi de fond 243 de la pièce-couvercle 24, de préférence elle vient de placer autour d'un bossage interne 247 faisant saillie au centre de la surface interne de la paroi de fond 243 de manière à opérer un maintien mécanique de l'extrémité arrière 233 du ressort cylindrique 23, comme expliqué ciavant pour le bossage 225. Le ressort 23 est donc maintenu par et entre les bossages interne 247 de la pièce-couvercle 24 et externe 225 de la pièce mobile 22.

Par ailleurs, comme illustré en Fig. 3, préférentiellement, des logements ou évidements, telles des encoches 226, sont aménagés dans la collerette 222 de la pièce mobile 22 et des guides axiaux 246, i.e. des rails de guidage ou analogue, sont aménagés axialement le long de la paroi interne de la pièce-couvercle 24. Les encoches 226 sont conformées pour loger lesdits guides axiaux 246 de sorte que ces guides axiaux 246 puissent guider les déplacements translatifs de la pièce mobile 22 dans la pièce-couvercle 24.

Autrement dit, grâce à ces encoches 226 et guides axiaux 246, la pièce mobile 22 peut mieux coulisser dans le volume interne de la pièce-couvercle 24, en particulier en fonction du taux de compression du ressort 23.

Dans le mode de réalisation de Fig. 3, on voit 4 encoches 226 aménagées dans la collerette 222 de la pièce mobile 22 coopérant avec 4 guides axiaux 246 correspondants aménagés axialement le long de la paroi interne de la pièce-couvercle 24.

Fig. 4 est une vue latérale et en coupe de la valve 2 de Fig. 2, dans sa position de « repos », c'est-à-dire libre de toute contrainte externe.

Dans ce cas, le ressort 23 est comprimé (zone 234) entre la collerette 222 de la pièce mobile 22 et le fond de la pièce-couvercle 24, et la pièce mobile 22 repousse alors le joint plat 21 contre le fond plat 205 du corps de valve 20 de manière à obtenir une étanchéité fluidique entre eux.

Dans cette position de « repos », aucun échange de gaz entre l'intérieur et l'extérieur de la cartouche 1 ne se produit au travers des valves 2, 3 étant donné qu'elles sont « fermées » étant donné que l'orifice 203 de chaque corps de valve 20 est alors obturé par la paroi de fond 223 de la pièce mobile 22.

La cartouche 1 est alors étanche et son volume interne 12 peut alors stocker de l'oxygène sous pression. Ainsi, avant utilisation, la cartouche 1 peut être remplie d'O₂ à une pression de quelques bars, par exemple de l'ordre d'au maximum 10 bar absolus, ce qui permet de répondre aux contraintes de logistiques, notamment d'acheminement et d'entreposage. L'introduction de l'O₂ dans l'enveloppe 11 peut se faire via une valve de remplissage (non montrée) agencée par exemple sur le couvercle 18 ou préférentiellement, via l'une des valves 2,3.

La présence de l'adsorbant 14 dans le volume interne 12 de la cartouche 1 permet de stocker une quantité d'oxygène très supérieure à celle que pourrait contenir ce récipient de gaz 1 en l'absence d'adsorbant.
Préférentiellement, on utilise en tant qu'adsorbant 14, une zéolite ayant une forte capacité d'adsorption d'oxygène, ce qui permet donc d'augmenter très notablement la capacité de stockage du gaz au sein de de la cartouche 1.

Par exemple, à une pression de 10 bar abs., une cartouche 1 de 660 ml de volume interne 12 peut contenir environ 400 g d'adsorbant 14 et, par conséquent, adsorber environ 12 L d'O₂, alors que l'espace interstitiel autour des particules d'adsorbant 14 définit un volume « vide » dans lequel des molécules d'O₂ vont également être comprimées et stockées, permettant ainsi d'augmenter la capacité totale à environ 15 L d'O₂ (à pression atmosphérique). A titre comparatif, en l'absence d'adsorbant 14, on ne pourrait y stocker qu'environ 6 L d'O₂, soit 2,5 fois moins.

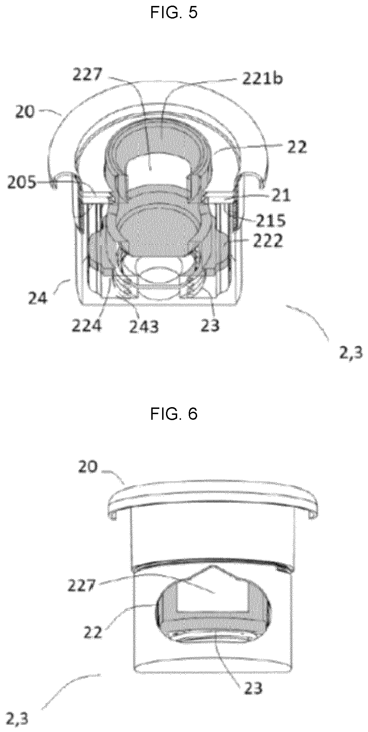

Fig. 5 et Fig. 6 représentent une configuration (vues partiellement tronquées) où ladite première valve 2 est en position « actionnée », c'est-à-dire lorsqu'une force extérieure vient s'appliquer sur la partie avant 221 de la pièce mobile 22, par exemple lors du raccordement d'un connecteur ou analogue, comme expliqué ci-après.

Dans ce cas, on voit que la pièce mobile 22 ne repousse plus le joint plat 21 contre le fond plat 205 du corps de valve 20 et que l'étanchéité fluidique est alors rompue, ce qui permet un passage de gaz au travers de ladite première valve 2.

Autrement dit, la pièce mobile 22 a subi une translation axiale vers l'intérieur de la cartouche 1, c'est-à-dire en direction de la pièce-couvercle 24, ce qui a pour conséquence de comprimer le ressort 23 vers le fond de la pièce-couvercle 24 tout en libérant le passage du gaz entre le joint plat 21 et le fond plat 205 du corps de valve 20.

La translation de la pièce mobile 22 en éloignement par rapport au corps de valve 20 engendre une perte de contact entre la face avant de la collerette 222 et le joint plat 21 et il se crée alors une communication fluidique entre l'extérieur et l'intérieur de la cartouche 1, via la première valve de contrôle 2, notamment au travers des ouvertures latérales 227 de la pièce mobile 22 et des ouvertures latérales 242 de la pièce-couvercle 24. Bien entendu, la seconde valve 3 fonctionne sur le même principe.

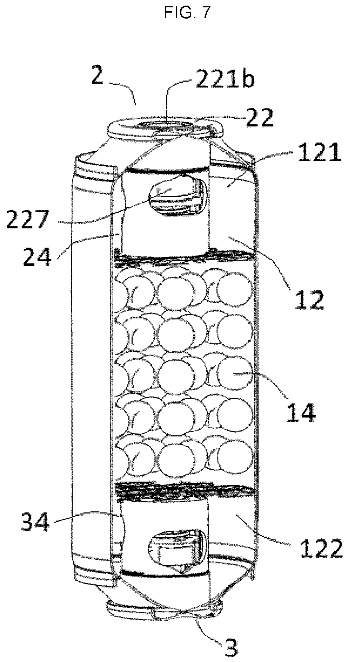

Fig. 7 montre la cartouche de stockage d'oxygène 1 avec ses deux valves de contrôle 2, 3 en position « actionnée », la structure et le fonctionnement de la deuxième valve de contrôle 3 étant identique à ceux de la première valve de contrôle 2.

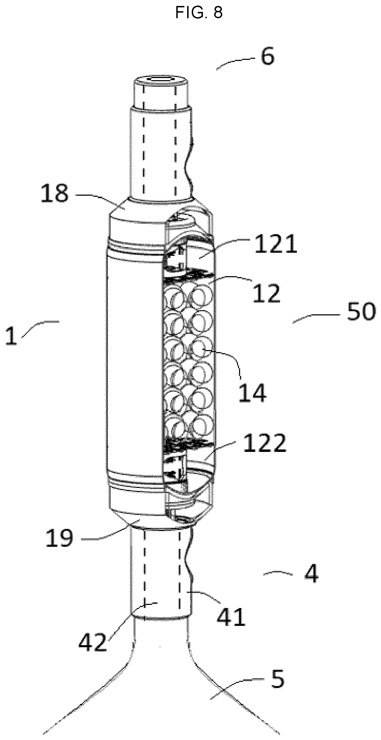

Les valves de contrôle 2, 3 sont dans une telle position « actionnée », lorsque la cartouche de stockage d'oxygène de Fig. 1 a été raccordée par un utilisateur à des dispositifs extérieurs, par exemple à, d'une part, un premier conduit de gaz 4 et, d'autre part, un second conduit de gaz 6, comme illustré sur Fig. 8, en formant ainsi un ensemble 50 de distribution d'oxygène de secours selon l'invention.

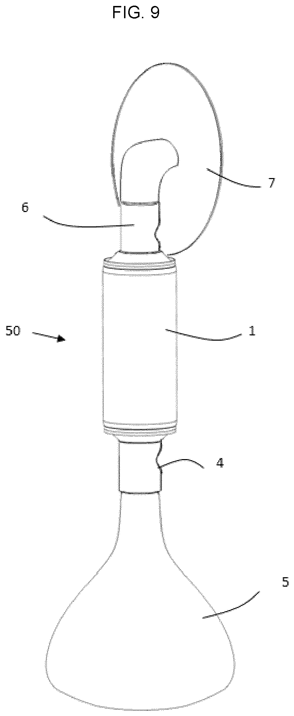

Comme on le voit en Fig. 8, le premier conduit 4 comprend une enveloppe cylindrique 41 disposant d'un conduit interne creux 42 ou lumen, ayant une forme de cylindre creux. Le premier conduit 4 est par ailleurs connecté fluidiquement un réservoir 5 déformable ou flexible, mieux visible en Fig. 9, vide de gaz, au moment du raccordement à la cartouche 1, mais pouvant d'être rempli, i.e. gonflé, d'un volume de gaz donné après raccordement du premier conduit 4 à la cartouche 1. Par exemple, le réservoir 5 peut être configuré pour collecter un volume de gaz supérieur ou égal au volume de gaz contenu dans le volume intérieur 12 de cartouche de gaz 1, i.e. O₂. Le réservoir 5 peut être formée d'une enveloppe souple en polymère.

Le premier conduit 4 est raccordé fluidiquement, de manière étanche, par exemple au fond 19 la cartouche 1, par exemple par clipsage, vissage ou autre, portant la seconde valve 3.

Le conduit interne 42 du premier conduit 4 va alors agir sur la pièce mobile 22 de la valve de contrôle 3 pour la repousser vers l'intérieur de la cartouche 1 de sorte que la seconde valve 3 se retrouve en position « actionnée », avec rupture de l'étanchéité au niveau du joint plat de de la valve de contrôle 3, comme expliqué ci-dessous.

Il s'opère alors un transfert d'O₂ depuis le volume intérieur 12 du récipient de gaz 1 vers le premier conduit 4 puis vers le réservoir 5 qui augmente progressivement en volume, c'est-à-dire se remplit d'oxygène. Une fois le réservoir 5 rempli, il s'établit un équilibre de pression entre le réservoir 5 flexible et la cartouche de gaz 1, cette dernière se retrouvant alors à pression atmosphérique (i.e. 1 atm).

En procédant de la même manière, l'utilisateur peut raccorder un second conduit 6 à la valve 2 équipant le couvercle 18, ce qui, là encore, va repousser la pièce mobile 22 pour faire passer la première valve 2 dans sa position « actionnée », comme décrit ci-dessus, et établir alors une connexion fluidique entre le conduit interne du second conduit 6, le volume interne 12, le conduit interne 42 du premier conduit 4 et ensuite, par extension, le réservoir 5.

Le second conduit 6 peut par exemple alimenter une interface respiratoire 7, tel un masque respiratoire qui peut être nasal, buccal ou facial (i.e. naso-buccal), c'est-à-dire que la cartouche 1 est alors prête à l'emploi par l'utilisateur, lequel peut inhaler de l'oxygène provenant de la cartouche 1 et/ou du réservoir 5, comme illustré en Fig. 9.

De préférence, on choisit un masque respiratoire 7 étanche, c'est-à-dire comprenant des moyens d'étanchéité sur le pourtour de l'ouverture par laquelle l'utilisateur respire, c'est-à-dire l'ouverture arrière du masque recevant le nez (masque nasal) ou le nez et la bouche de l'utilisateur (masque facial), par exemple des moyens d'étanchéité de type coussin flexible ou analogue venant au contact des zones du visage de l'utilisateur entourant son nez et/ou sa bouche, afin d'y créer une étanchéité fluidique.

Ainsi, lors d'une phase inspiratoire de l'utilisateur, une partie de l'O₂ contenu dans le réservoir flexible 5, ressort de celui-ci puis transite successivement dans le conduit interne 42 du premier conduit 4, le volume intérieur 12 de la cartouche 1 au travers du lit d'adsorbant 14 et ensuite ressortir du volume intérieur 12 via le second conduit 6 et atteindre enfin le masque respiratoire 7 porté l'utilisateur.

A l'inverse, lors d'une phase expiratoire de l'utilisateur, une partie de l'O₂ inhalé (env. 5% vol.) a été substituée par du CO₂ et le gaz exhalé par l'utilisateur contient donc du CO₂, de la vapeur d'eau et une haute concentration d'O₂, c'est-à-dire plus de 90 % en volume. Le gaz expiré va suivre le chemin inverse à celui suivi par l'oxygène inhalé, à savoir emprunter le second conduit 6, traverser la première valve 2 pour atteindre alors le volume intérieur 12 de la cartouche 1, et transiter ensuite successivement au travers du lit d'adsorbant 14, puis de la seconde valve 3 et du premier conduit 4 pour atteindre enfin le réservoir déformable 5.

Une caractéristique de l'adsorbant 14 choisi, telles des particules de zéolite, est que sa capacité d'adsorption diffère selon les molécules considérées. Ainsi, la capacité d'adsorption de l'O₂ est relativement faible par rapport à la capacité d'adsorption du CO₂ ou de la vapeur d'eau, typiquement de 50 à 100 fois plus importante. Pour une concentration de 5% de CO₂, c'est-à-dire 5 kPa en pression absolue, la capacité d'adsorption d'une zéolite, par exemple de type Zeochem Z10-ZZ ou 5A, excède aisément 1.5 moles par kg, présentant ainsi une capacité de stockage en volume supérieure au volume total d'O₂ initialement présent dans la cartouche de gaz 1, mesurée à 10 bar. Cette capacité est encore plus importante pour la vapeur d'eau.

Dès lors, lorsque le gaz expiré par l'utilisateur traverse l'adsorbant 14, les molécules de CO₂ et la vapeur d'eau vont être adsorbées de sorte que le gaz est débarrassé de la quasi-totalité du CO₂ (et de la vapeur d'eau) qu'il contient, c'est-à-dire qu'il s'agit d'oxygène quasi-pur. Cet oxygène gazeux exempt de CO₂, qui retourne dans le réservoir déformable 5 peut ensuite être inspiré à nouveau par l'utilisateur, lors d'une phase inspiratoire suivante.

A titre indicatif, la consommation métabolique en oxygène d'un individu est au maximum de 0.5 L/min, ce qui implique qu'après 10 minutes d'inhalation/expiration répétées, le réservoir 5 contient environ 5L d'O₂ en moins, dans les conditions susmentionnées, à savoir une pression de 10 bar et un volume du récipient de 660 ml. Ceci permet de subvenir aux besoins respiratoires d'un utilisateur pendant plusieurs dizaines de minutes.

Bien entendu, on peut utiliser plusieurs adsorbants différents, par exemple plusieurs lits successifs de différents adsorbants.

Un tel ensemble 50 de distribution d'oxygène de secours comprenant une cartouche 1 de stockage d'oxygène portative selon l'invention raccordée, d'une part, à un réservoir 5 et, d'autre part, à une interface respiratoire 7, tel un masque respiratoire, peut être utilisé pour fournir de l'oxygène à une personne, pendant un temps suffisant, dans une situation particulière engendrant une atmosphère néfaste, hypoxique et/ou chargée de composés délétères, en particulier en présence de fumées générées par un incendie.

L'ensemble de distribution d'oxygène de secours 50 selon l'invention est portatif, c'est-à-dire qu'il peut être porté facilement par un utilisateur, étant donné que ses dimensions et son poids permettent à une personne de le soulever, le transporter et l'utiliser facilement et sans effort physique particulier.

## Revendications

1. Ensemble (50) de distribution d'oxygène de secours comprenant une cartouche (1) de stockage d'oxygène comprenant un corps tubulaire (13) fermé à deux extrémités opposées (13A, 13B) et délimitant un volume interne (12) servant au stockage de l'oxygène, dans laquelle :
- au moins une valve de contrôle de flux gazeux (2, 3) est agencée à chacune desdites extrémités opposées (13A, 13B), et
- le volume interne (12) comprend une chambre centrale (124) contenant au moins un lit d'adsorbant (14), une première chambre de recueil de gaz (121) et une seconde chambre de recueil de gaz (122), la chambre centrale (124) étant située entre les première et seconde chambres de recueil de gaz (121, 122),
**caractérisé en ce que** la cartouche (1) est raccordée fluidiquement à un réservoir de gaz (5) et à une interface respiratoire (7).

2. Ensemble selon la revendication 1, **caractérisé en ce que** la chambre centrale (124) de la cartouche (1) est séparée de la première et de la seconde chambre de recueil de gaz (121, 122) par une première et une seconde structure de tamis (15, 16), respectivement.

3. Ensemble selon la revendication 2, **caractérisé en ce que** le corps tubulaire (13) de la cartouche (1) a une section interne et les structures de tamis (15, 16) sont agencées radialement dans le volume interne (12), i.e. perpendiculairement à l'axe principal (XX), de manière à s'étendre sur toute la section interne du corps tubulaire (13).

4. Ensemble selon l'une des revendications 2 ou 3, **caractérisé en ce que** les structures de tamis (15, 16) sont configurées pour maintenir le lit d'adsorbant (14).

5. Ensemble selon l'une des revendications 1 ou 4, **caractérisé en ce que** le lit d'adsorbant (14) contient des particules de zéolite, de préférence une zéolite 13X ou 5A présentant une capacité d'adsorption pour l'oxygène d'au moins 3 ml/g à 1 bar et 20°C.

6. Ensemble selon la revendication 1, **caractérisé en ce que** chaque valve de contrôle (2, 3) de la cartouche (1) comprend un corps de valve (20), un moyen d'étanchéité annulaire (21), un élément mobile (22) et un moyen élastique (23) coopérant les uns avec les autres pour contrôler le passage de gaz au travers de chaque valve de contrôle (2, 3).

7. Ensemble selon la revendication 6, **caractérisé en ce que** le moyen élastique (23) repousse l'élément mobile (22) contre le moyen d'étanchéité annulaire (21), de préférence le moyen élastique (23) est un ressort à spires cylindrique et le moyen d'étanchéité annulaire (21) est un joint torique plat.

8. Ensemble selon l'une des revendications 6 ou 7, **caractérisé en ce que** le corps de valve (20) comprend un fond annulaire (205) présentant un évidement central (203), le moyen d'étanchéité annulaire (21) étant agencé sur ledit fond annulaire (205), coaxialement audit évidement central (203).

9. Ensemble selon l'une des revendications 6 à 8, **caractérisé en ce que** l'élément mobile (22) de la cartouche (1) comprend une collerette (222) coopérant avec le moyen d'étanchéité annulaire (21) pour assurer une étanchéité fluidique et empêcher toute circulation de gaz au travers de l'évidement central (203) du corps de valve (20).

10. Ensemble (50) selon à la revendication 1, **caractérisé en ce que** l'interface respiratoire (7) est un masque respiratoire.

11. Ensemble selon la revendication 1, **caractérisé en ce que** l'interface respiratoire (7) et le réservoir de gaz (5) comprennent chacun un conduit de raccordement (4, 6), en particulier un premier conduit et un second conduit.

12. Ensemble selon la revendication 11, **caractérisé en ce que** les conduits de raccordement (4, 6) de l'interface respiratoire (7) et du réservoir de gaz (5) coopèrent avec des moyens de raccordement portés par la cartouche (1) pour assurer un raccordement de l'interface respiratoire (7) et du réservoir de gaz (5) à la cartouche (1).

13. Ensemble selon la revendication 1, **caractérisé en ce que** :
- le réservoir (5) comprend une enveloppe souple en polymère et/ou
- la cartouche (1) a un volume intérieur compris entre 200 et 1000 ml, de préférence compris entre 300 ml à 900 ml (équiv. en eau).

14. Ensemble selon la revendication 1, **caractérisé en ce que** le corps tubulaire (13) est fermé à ses deux extrémités opposées (13A, 13B) par un premier et un second couvercle (18, 19), les valve de contrôle de flux gazeux (2, 3) étant agencées sur lesdits premier et second couvercles (18, 19).

15. Utilisation d'un ensemble de distribution d'oxygène de secours (50) comprenant une cartouche de gaz (1) selon l'une des revendications précédentes, pour stocker de l'oxygène gazeux.

## Patentansprüche

1. Notsauerstoffabgabeanordnung (50), welche eine Sauerstoffspeicherpatrone (1) umfasst, die einen rohrförmigen Körper (13) umfasst, der an zwei gegenüberliegenden Enden (13A, 13B) verschlossen ist und ein Innenvolumen (12) begrenzt, das zur Speicherung des Sauerstoffs dient, wobei:
- mindestens ein Gasfluss-Steuerventil (2, 3) an jedem der gegenüberliegenden Enden (13A, 13B) angeordnet ist und
- das Innenvolumen (12) eine zentrale Kammer (124), die mindestens ein Adsorberbett (14) enthält, eine erste Gassammelkammer (121) und eine zweite Gassammelkammer (122) umfasst, wobei sich die zentrale Kammer (124) zwischen der ersten und der zweiten Gassammelkammer (121, 122) befindet,
**dadurch gekennzeichnet, dass** die Patrone (1) fluidisch an einen Gasbehälter (5) und an eine Beatmungsschnittstelle (7) angeschlossen ist.

2. Anordnung nach Anspruch 1, **dadurch gekennzeichnet, dass** die zentrale Kammer (124) der Patrone (1) von der ersten und der zweiten Gassammelkammer (121, 122) durch eine erste bzw. eine zweite Siebstruktur (15, 16) getrennt ist.

3. Anordnung nach Anspruch 2, **dadurch gekennzeichnet, dass** der rohrförmige Körper (13) der Patrone (1) einen Innenquerschnitt aufweist und die Siebstrukturen (15, 16) in dem Innenvolumen (12) radial angeordnet sind, d. h. senkrecht zu der Hauptachse (XX), sodass sie sich über den gesamten Innenquerschnitt des rohrförmigen Körpers (13) erstrecken.

4. Anordnung nach einem der Ansprüche 2 oder 3, **dadurch gekennzeichnet, dass** die Siebstrukturen (15, 16) dazu konfiguriert sind, das Adsorberbett (14) zu halten.

5. Anordnung nach einem der Ansprüche 1 oder 4, **dadurch gekennzeichnet, dass** das Adsorberbett (14) Zeolithpartikel enthält, vorzugsweise ein Zeolith 13X oder 5A, das bei 1 bar und 20 °C eine Adsorptionskapazität für Sauerstoff von mindestens 3 ml/g aufweist.

6. Anordnung nach Anspruch 1, **dadurch gekennzeichnet, dass** jedes Steuerventil (2, 3) der Patrone (1) einen Ventilkörper (20), ein ringförmiges Dichtmittel (21), ein bewegliches Element (22) und ein elastisches Mittel (23) umfasst, die untereinander zusammenwirken, um den Durchfluss von Gas durch das jeweilige Steuerventil (2, 3) zu steuern.

7. Anordnung nach Anspruch 6, **dadurch gekennzeichnet, dass** das elastische Mittel (23) das bewegliche Element (22) gegen das ringförmige Dichtmittel (21) zurückdrückt, wobei vorzugsweise das elastische Mittel (23) eine zylindrische Spiralfeder ist und das ringförmige Dichtmittel (21) ein flacher O-Ring ist.

8. Anordnung nach einem der Ansprüche 6 oder 7, **dadurch gekennzeichnet, dass** der Ventilkörper (20) einen ringförmigen Boden (205) umfasst, der eine zentrale Aussparung (203) aufweist, wobei das ringförmige Dichtmittel (21) auf dem ringförmigen Boden (205) angeordnet ist, koaxial mit der zentralen Aussparung (203) .

9. Anordnung nach einem der Ansprüche 6 bis 8, **dadurch gekennzeichnet, dass** das bewegliche Element (22) der Patrone (1) einen Flansch (222) umfasst, der mit dem ringförmigen Dichtmittel (21) zusammenwirkt, um eine Fluiddichtheit sicherzustellen und jedes Strömen von Gas durch die zentrale Aussparung (203) des Ventilkörpers (20) hindurch zu verhindern.

10. Anordnung (50) nach Anspruch 1, **dadurch gekennzeichnet, dass** die Beatmungsschnittstelle (7) eine Atemmaske ist.

11. Anordnung nach Anspruch 1, **dadurch gekennzeichnet, dass** die Beatmungsschnittstelle (7) und der Gasbehälter (5) jeweils eine Anschlussleitung (4, 6) umfassen, insbesondere eine erste Leitung und eine zweite Leitung.

12. Anordnung nach Anspruch 11, **dadurch gekennzeichnet, dass** die Anschlussleitungen (4, 6) der Beatmungsschnittstelle (7) und des Gasbehälters (5) mit Anschlussmitteln zusammenwirken, die von der Patrone (1) getragen werden, um einen Anschluss der Beatmungsschnittstelle (7) und des Gasbehälters (5) an die Patrone (1) sicherzustellen.

13. Anordnung nach Anspruch 1, **dadurch gekennzeichnet, dass**:
- der Behälter (5) eine flexible Hülle aus Polymer umfasst und/oder
- die Patrone (1) ein Innenvolumen zwischen 200 und 1000 ml aufweist, vorzugsweise zwischen 300 ml und 900 ml (Wasseräquivalent).

14. Anordnung nach Anspruch 1, **dadurch gekennzeichnet, dass** der rohrförmige Körper (13) an seinen zwei gegenüberliegenden Enden (13A, 13B) durch einen ersten und einen zweiten Deckel (18, 19) verschlossen ist, wobei die Gasfluss-Steuerventile (2, 3) an dem ersten und zweiten Deckel (18, 19) angeordnet sind.

15. Verwendung einer Notsauerstoffabgabeanordnung (50), welche eine Gaspatrone (1) umfasst, nach einem der vorhergehenden Ansprüche, zum Speichern von gasförmigem Sauerstoff.

## Claims

1. Assembly (50) for emergency oxygen distribution, comprising an oxygen storage cartridge (1) comprising a tubular body (13) closed at two opposite ends (13A, 13B) and delimiting an internal volume (12) that serves to store oxygen, in which:
- at least one gas flow control valve (2, 3) is arranged at each of said opposite ends (13A, 13B), and
- the internal volume (12) comprises a central chamber (124) containing at least one adsorbent bed (14), a first gas collection chamber (121) and a second gas collection chamber (122), the central chamber (124) being located between the first and second gas collection chambers (121, 122),
**characterized in that** the cartridge (1) is fluidically connected to a gas reservoir (5) and to a respiratory interface (7).

2. Assembly according to Claim 1, **characterized in that** the central chamber (124) of the cartridge (1) is separated from the first and second gas collection chambers (121, 122) by first and second screen structures (15, 16), respectively.

3. Assembly according to Claim 2, **characterized in that** the tubular body (13) of the cartridge (1) has an internal cross section and the screen structures (15, 16) are arranged radially in the internal volume (12), i.e. perpendicular to the main axis (XX), so as to extend over the entire internal cross section of the tubular body (13) .

4. Assembly according to either of Claims 2 and 3, **characterized in that** the screen structures (15, 16) are configured to hold the adsorbent bed (14).

5. Assembly according to either of Claims 1 and 4, **characterized in that** the adsorbent bed (14) contains zeolite particles, preferably zeolite 13X or 5A having an oxygen adsorption capacity of at least 3 ml/g at 1 bar and 20°C.

6. Assembly according to Claim 1, **characterized in that** each control valve (2, 3) of the cartridge (1) comprises a valve body (20), an annular sealing means (21), a movable element (22) and resilient means (23) cooperating with one another to control the passage of gas through each control valve (2, 3).

7. Assembly according to Claim 6, **characterized in that** the resilient means (23) urges the movable element (22) against the annular sealing means (21), and preferably the resilient means (23) is a cylindrical coil spring and the annular sealing means (21) is a flat O-ring.

8. Assembly according to either of Claims 6 and 7, **characterized in that** the valve body (20) comprises an annular bottom (205) having a central recess (203), the annular sealing means (21) being arranged on said annular bottom (205), coaxially with respect to said central recess (203).

9. Assembly according to one of Claims 6 to 8, **characterized in that** the movable element (22) of the cartridge (1) comprises a flange (222) cooperating with the annular sealing means (21) in order to ensure fluid sealing and to prevent any circulation of gas through the central recess (203) of the valve body (20).

10. Assembly (50) according to Claim 1, **characterized in that** the respiratory interface (7) is a breathing mask.

11. Assembly according to Claim 1, **characterized in that** the respiratory interface (7) and the gas reservoir (5) each comprise a connecting conduit (4, 6), in particular a first conduit and a second conduit.

12. Assembly according to Claim 11, **characterized in that** the connecting conduits (4, 6) of the respiratory interface (7) and of the gas reservoir (5) cooperate with connection means carried by the cartridge (1) in order to ensure a connection of the respiratory interface (7) and the gas reservoir (5) to the cartridge (1).

13. Assembly according to Claim 1, **characterized in that**:
- the reservoir (5) comprises a flexible envelope made of polymer, and/or
- the cartridge (1) has an inner volume of between 200 and 1000 ml, preferably of between 300 ml and 900 ml (equiv. in water).

14. Assembly according to Claim 1, **characterized in that** the tubular body (13) is closed at its two opposite ends (13A, 13B) by first and second covers (18, 19), the gas flow control valves (2, 3) being arranged on said first and second covers (18, 19).

15. Use of an assembly (50) for emergency oxygen distribution comprising a gas cartridge (1) according to any one of the preceding claims, for storing gaseous oxygen.
